# EUROPÄISCHE PATENTSCHRIFT

(11) **EP 1 247 869 B1**
(45) Veröffentlichungstag und Bekanntmachung des Hinweises auf die Patenterteilung: **04.10.2006**
(21) Anmeldenummer: 02007262.5
(22) Anmeldetag: 28.03.2002
(51) Int. Cl.: C12P 13/04, C12N 9/88

(54) **Verfahren zur Herstellung von nicht-proteinogenen L-Aminosäuren**
Process for the production of non-proteinogenic amino acids
Procédé de fabrication d' acides aminés non-protéinogènes

(30) Priorität: 04.04.2001 DE 10116881; 03.05.2001 DE 10121515
(43) Veröffentlichungstag der Anmeldung: 09.10.2002
(73) Patentinhaber: Consortium für elektrochemische Industrie GmbH, 81379 München (DE)
(72) Erfinder: Maier, Thomas, Dr., 85221 Dachau (DE); Gaebert, Carsten, Dr., 81371 München (DE)
(74) Vertreter: Potten, Holger

(56) Entgegenhaltungen:
- EP-A- 0 323 068
- EP-A- 1 191 106
- PATENT ABSTRACTS OF JAPAN vol. 2000, no. 20, 10. Juli 2001 (2001-07-10) -& JP 2001 061489 A (DAI ICHI PURE CHEM CO LTD), 13. März 2001 (2001-03-13)
- NOJI MASAAKI ET AL: "Evidence for identity of beta-pyrazolealanine synthase with cysteine synthase in watermelon: Formation of beta-pyrazolealanine by cloned cysteine synthase in vitro and in vivo." BIOCHEMICAL AND BIOPHYSICAL RESEARCH COMMUNICATIONS, Bd. 197, Nr. 3, 1993, Seiten 1111-1117, XP002202953 ISSN: 0006-291X
- IKEGAMI FUMIO ET AL: "Enzymic synthesis of non-protein beta-substituted alanines and some higher homologues in plants." PHYTOCHEMISTRY (OXFORD), Bd. 35, Nr. 5, 1994, Seiten 1089-1104, XP008004952 ISSN: 0031-9422
- SAITO ET AL: "production of plant non-protein amino acids by recombinant enzymes of sequential biosynthetic reactions in bacteria" BIOLOGICAL AND PHARMACEUTICAL BULLETIN, TOKYO, JP, Bd. 20, Nr. 1, Januar 1997 (1997-01), Seiten 47-53, XP002122799 ISSN: 0918-6158
- PATENT ABSTRACTS OF JAPAN vol. 008, no. 019 (C-207), 26. Januar 1984 (1984-01-26) -& JP 58 187198 A (SHOWA DENKO KK), 1. November 1983 (1983-11-01)

## Beschreibung

Die Erfindung betrifft ein Verfahren zur Herstellung von nicht-proteinogenen L-Aminosäuren durch enzymatische Biotransformation.

Nicht-proteinogene Aminosäuren sind Aminosäuren, die in der Natur nicht als Bausteine für die Proteinbiosynthese verwendet werden und dadurch klar von den 20 proteinogenen Aminosäuren abzugrenzen sind. Im Sinne der vorliegenden Erfindung wird die sehr seltene Aminosäure L-Selenocystein, die durchaus in Proteinen vorkommt, den nicht-proteinogenen Aminosäuren zugerechnet.

Nicht-proteinogene Aminosäuren stellen interessante Verbindungen z.B. für die Herstellung von Pharma- und Agrowirkstoffen dar. Sie können als Wirkstoff oder als Teil eines Wirkstoffs in einer Art molekularer Mimikry die Struktur von natürlichen Aminosäuren imitieren und dadurch zum Beispiel bei Rezeptor-Interaktionen eine Modulation der natürlichen Reaktion bewirken. Zudem können sie ganz allgemein als chirale Verbindungen im Rahmen des "chiral pool" als Synthesebausteine dienen.

Bisherige Herstellverfahren für nicht-proteinogene Aminosäuren in enantiomerenreiner Form basieren meist auf aufwändigen Synthesen, die zudem meist nur Zugang zu einer bestimmten Verbindung erlauben. Nur wenige Verfahren ermöglichen durch den einfachen Austausch eines Eduktes die Herstellung verschiedener Verbindungen. In den meisten Fällen handelt es sich um chemische Synthesen, die ihrerseits meist schon von chiralen Bausteinen ausgehen. Andere Verfahren kombinieren die chemische Synthese von Razematen mit einer Razematspaltung, die häufig enzymatisch durchgeführt wird.

Daneben sind auch einige enzymatische Verfahren beschrieben, die von prochiralen Verbindungen ausgehen und die stereoselektive Synthese von nicht-proteinogenen Aminosäuren ermöglichen.

So können mit Hilfe von Transaminasen aus α-Ketosäuren mit L-Glutaminsäure als Amino-Donor verschiedene nicht-proteinogene Aminosäuren hergestellt werden(Taylor et al. 1998, TIBTECH 16: 412-418). Ein weiteres Beispiel ist die Synthese von L-tert-Leucin mit Hilfe von Leucin-Dehydrogenase (Drauz 1997, Chimia 51: 310 -314).

Ein besonders einfaches Verfahren zur Herstellung von nicht-proteinogenen Aminosäuren durch direkte Fermentation von Mikroorganismen ist in der Patentanmeldung DE 10046934 (angemeldet am 21.09.2000 vom gleichen Anmelder) beschrieben. Hierbei kommen Organismen zum Einsatz, die einen deregulierten Cystein-Stoffwechsel aufweisen und deshalb einen hohen Spiegel an O-Acetyl-L-Serin bereitstellen. Diese Verbindung dient im Cystein-Stoffwechsel als biosynthetischer Vorläufer von L-Cystein. Letzteres entsteht durch Substitution der Acetat-Gruppe an der β-Position gegen einen Thiolrest. Diese als β-Substitution bezeichnete Reaktion wird von Enzymen der Klasse O-Acetyl-L-Serin-Sulfhydrylasen [EC 4.2.99.8] katalysiert. Durch Zufütterung von nukleophilen Substanzen bestimmter Verbindungsklassen (Thiole, Azole bzw. Isoxazolinone) während der Fermentation gehen diese in die β-Substitution ein und es wird die Produktion von nicht-proteinogenen L-Aminosäuren erreicht. Die Struktur der jeweiligen Reste der hergestellten Aminosäuren werden somit durch die zugeführte, nukleophile Verbindung diktiert.

Ein Problem bei diesem Verfahren ist, dass die zugefütterten nukleophilen Verbindungen nicht zu hoch dosiert werden dürfen, da andernfalls durch die Verbindung selbst oder durch die resultierende Aminosäure toxische Effekte auf den Stoffwechsel der Mikroorganismen hervorgerufen werden können. Dies trifft insbesondere für viele Thiolverbindungen zu, da sie als redoxaktive Substanzen in höheren Konzentrationen Toxizität besitzen. Zudem ist der Einsatz von Thiolverbindungen in fermentativen Verfahren sehr problematisch, weil sie bei intensiver Belüftung im Fermenter zur Oxidation neigen und ohne technische Vorkehrungen eine signifikante Geruchsbelästigung hervorrufen. Auch ein Zufüttern von Azid oder Cyanid von denen bekannt ist, dass sie in die β-Substitution mit O-Acetyl-L-Serin-Sulfhydrylasen eingehen (Flint et al., 1996, J. Biol. Chem. 271:16053-16067), ist aufgrund ihrer hohen Toxizität nicht möglich.

Aufgabe der vorliegenden Erfindung war es, ein Verfahren zur Herstellung von nicht-proteinogenen L-Aminosäuren bereitzustellen, das einen hoch dosierten Einsatz von nukleophilen Verbindungen - insbesondere auch von toxischen Verbindungen - ermöglicht.

Die Aufgabe wird gelöst durch ein Verfahren zur Herstellung einer nicht-proteinogenen L-Aminosäure mittels einer enzymatischen Biotransformation bei dem O-Acetyl-L-Serin mit einer nukleophilen Verbindung unter Katalyse einer O-Acetyl-L-Serin-Sulfhydrylase zu einer nicht-proteinogenen L-Aminosäure umgesetzt wird, dadurch gekennzeichnet, daß die Biotransformation unter Einsatz von ruhenden Mikroorganismenzellen, die eine O-Acetyl-L-Serin-Sulfhydrylase Aktivität besitzen, erfolgt und das Verfahren bei einem pH-Wert im Bereich zwischen pH 6,0 und 6,99 durchgeführt wird.

Durch dieses Verfahren wird die Synthese einer Vielzahl von nicht-proteinogenen, enantiomerenreinen und zum Teil neuartigen L-Aminosäuren im industriellen Maßstab möglich.

O-Acetyl-L-Serin-Sulfhydrylasen sind bekannt. Sie sind bisher aus verschiedensten Pflanzen und Mikroorganismen isoliert worden. Am besten untersucht sind die entsprechenden bakteriellen Enzyme von Salmonella typhimurium. In diesem Organismus existieren zwei O-Acetyl-L-Serin-Sulfhydrylase-Enzyme, die mit OASS-A bzw. OASS-B bezeichnet werden. Die zugehörigen Gene sind ebenfalls bekannt und tragen die Bezeichnung cysK bzw. cysM. Obwohl beide Enzyme einen sehr ähnlichen Reaktionsmechanismus besitzen, beträgt die Identität auf Basis der Aminosäuresequenz nur 45 %.

Für OASS-B (CysM) wurde beschrieben, dass sie im Gegensatz zu OASS-A (CysK) in der Lage ist, eine Reaktion von O-Acetyl-L-Serin mit Thiosulfat zu S-Sulfocystein zu katalysieren. Diese Reaktion spielt beim Wachstum der Bakterien mit Thiosulfat als einziger Schwefelquelle eine wichtige Rolle.

Sequenzvergleiche von O-Acetyl-L-Serin-Sulfhydrylase-Genen aus verschiedenen Organismen zeigen zudem, dass es zwei phylogenetische Gruppen gibt (Kitabatake et al., 2000, J. Bacteriol. 182: 143-145). CysK von Salmonella typhimurium und Escherichia coli bilden eine große Gruppe zusammen mit den O-Acetyl-L-Serin-Sulfhydrylasen aus anderen Eubacteria, aus methanogenen Archaea und aus Pflanzen. CysM von Salmonella typhimurium und Escherichia coli liegen dagegen in einer sehr kleinen Verwandtschaftsgruppe mit den O-Acetyl-L-Serin-Sulfhydrylasen aus hyperthermophilen Archaea (z.B. Pyrococcus, Sulfolobus, Thermoplasma).

O-Acetyl-L-Serin-Sulfhydrylasen im Sinne der vorliegenden Erfindung sind dadurch gekennzeichnet, dass sie die Synthese von L-Cystein aus O-Acetyl-L-Serin und Sulfid katalysieren können. Sowohl CysM- als auch CysK-verwandte Enzyme sind daher O-Acetyl-L-Serin-Sulfhydrylasen im Sinne der vorliegenden Erfindung.

Obwohl einzelne Publikationen zeigen, dass O-Acetyl-L-Serin-Sulfhydrylasen aus der CysK-Gruppe ein relativ breites Substratspektrum aufweisen (Ikegami & Murakoshi, 1994, Phytochemistry 35: 1089-1104; Flint et al., 1996, J. Biol. Chem. 271:16053-16067), wurde bisher die Möglichkeit des technischen Einsatzes von O-Acetyl-L-Serin-Sulfhydrylasen zur Produktion von nicht-proteinogenen Aminosäuren nicht in Betracht gezogen.

Der entscheidende Grund, der einer technischen Realisierung der enzymatischen Herstellung von nicht-proteinogenen Aminosäuren mit O-Acetyl-L-Serin-Sulfhydrylasen bisher entgegenstand, liegt in der Instabilität von O-Acetyl-L-Serin gerade in dem pH-Bereich, der dem Aktivitätsbereich der O-Acetyl-L-Serin-Sulfhydrylasen entspricht.

O-Acetyl-L-Serin isomerisiert in Abhängigkeit vom pH-Wert zu N-Acetyl-L-Serin. Die Reaktion ist irreversibel und beispielsweise bei einem pH-Wert von 7,6 mit Raten von 1 % x min⁻¹ auch extrem schnell. Die Reaktionsgeschwindigkeit sinkt mit abnehmendem pH-Wert, so daß die Verbindung z.B. bei pH 4,0 stabil ist. Der Mechanismus der Reaktion beruht auf einem intramolekularen, nukleophilen Angriff der deprotonierten Aminogruppe am Carbonyl-Kohlenstoff des Acyl-Rests (Tai et al. 1995, Biochemistry 34: 12311-12322).

Das pH-Optimum von O-Acetyl-L-Serin-Sulfhydrylasen liegt dagegen im Bereich von pH 8,0 (Ikegami & Murakoshi, 1994, Phytochemistry 35: 1089-1104; Tai et al. 1995, Biochemistry 34: 12311-12322) und somit in einem Bereich der bezüglich der O-Acetyl-Serin-Isomerisierung sehr unvorteilhaft ist.

Aus diesem Grund schlagen Ikegami & Murakoshi eine biomimetische Synthese von nicht-proteinogen Aminosäuren mit O-Acetyl-L-Serin, Nukleophil, Pyridoxalphosphat und Metallionen (bevorzugt Ga²⁺) vor. Diese Reaktion ist in einem pH-Bereich von 3,5 - 5,5 möglich und gewährleistet somit die Stabilität von O-Acetyl-L-Serin. Mit maximalen Ausbeuten < 45 % ist die Effizienz dieser Methode jedoch nicht sehr hoch. Ein großer Nachteil gegenüber der enzymatischen Synthese ist das Fehlen von Enantioselektivität.

Eine weitere Aufgabe die durch das erfindungsgemäße Verfahren gelöst wird, war es daher, ein enzymatisches Verfahren zur Herstellung von enantiomerenreinen nicht-proteinogenen Aminosäuren bereitzustellen, das trotz der Inkompatibilität von O-Acetyl-L-Serin-Stabilität und Enzymaktivitätsoptimum einen effizienten enzymatischen Umsatz(>> 45 %) bei geringer Isomerisierung gewährleistet.

Die Aufgabe wurde durch das erfindungsgemäße Verfahren gelöst, da es sich vorzugsweise dadurch auszeichnet, dass
- die Reaktion unterhalb des pH-Optimums der O-Acetyl-L-Serin-Sulfhydrylasen durchgeführt wird, und
- das Enzym in ausreichend hoher Dosierung eingesetzt wird.

Der pH-Wert wird bevorzugt durch eine aktive pH-Kontrolle konstant gehalten, um auch der stöchiometrischen Bildung von Essigsäure entgegen zu wirken. Die aktive pH-Kontrolle wird vorzugsweise durch eine Meß- und Regeleinheit bewerkstelligt, die bei Abweichung des pH-Wertes vom Sollwert durch Dosierung einer Lauge oder Säure den gewünschten pH-Wert wieder einstellt.

Die bisher in der Literatur beschriebenen Reaktionen zur Synthese von nicht-proteinogenen Aminosäuren mit O-Acetyl-L-Serin-Sulfhydrylasen wurden entgegen dem erfindungsgemäßen Vorgehen im Bereich des pH-Optimums der O-Acetyl-L-Serin-Sulfhydrylasen, ohne aktive pH-Regulierung und lediglich im analytischen Massstab durchgeführt. Zudem wurden ausnahmslos Enzyme der phylogenetischen Gruppe der CysK-Enzyme verwendet.

Vorzugsweise wird im erfindungsgemäßen Verfahren als O-Acetyl-L-Serin-Sulfhydrylase CysM eingesetzt.

Eine ausreichend hohe Dosierung des Enzyms gewährleistet, dass auch außerhalb des pH-Optimums der Enzymreaktion ein ausreichender Umsatz stattfindet. Eine solche Enzymkonzentration ist für das Verfahren vorzugsweise dann gegeben, wenn die Volumenaktivität der O-Acetyl-L-Serin-Sulfhydrylase A_{Cys} mindestens 2 Units / ml im Ansatz beträgt. Besonders bevorzugt beträgt die Aktivität 2 - 200 Units / ml. Die Aktivitätsbestimmung erfolgt nach dem in Beispiel 3 beschriebenen Test.

Es konnte im Zuge der vorliegenden Erfindung beobachtet werden, dass auch O-Acetyl-Serin-Sulfhydrylasen aus der phylogenetischen Gruppe der CysM-Enzyme hervorragende Enzyme für die Herstellung von nicht-proteinogenen Aminosäuren darstellen. Das Spektrum der als Substrat geeigneten Nukleophile ist überraschenderweise sogar noch breiter als dasjenige der CysK-Enzyme.

In einer besonders bevorzugten Ausführungsform der Erfindung wird die Enzymreaktion als kontinuierliches Verfahren betrieben. Hierbei wird während der Reaktion permanent O-Acetyl-L-Serin, O-Acetyl-L-Serin-Sulfhydrylase und Nukleophil zudosiert und gleichzeitig dem Ansatz eine Lösung enthaltend die nicht-proteinogenen L-Aminosäure (Produktlösung) entnommen. Letzteres geschieht vorzugsweise derart, dass das Volumen im Reaktionsansatz gleichbleibend ist. Der besondere Vorteil dieser Vorgehensweise ist, dass ein Fließgleichgewicht eingestellt wird, so dass eine permanent niedrige Konzentration von O-Acetyl-L-Serin im Ansatz vorliegt und somit die Isomerisierung vermindert wird. Vorzugsweise wird eine O-Acetyl-L-Serin-Konzentration von < 1,0 g/l im Ansatz eingestellt. Dieser Wert kann durch Variation der mittleren Verweilzeit der Lösung im Reaktionsansatz gesteuert werden. Die Vorlage an O-Acetyl-L-Serin für die kontinuierliche Umsetzung wird unter saurem pH-Wert gehalten , vorzugsweise bei pH 4 - 5, um eine ausreichende Stabilität zu gewährleisten.

O-Acetyl-L-Serin war bisher nur aus chemischer Synthese durch Acetylierung von L-Serin zugänglich und aufgrund der hohen L-Serin-Preise teuer. Die am 15. Feb. 2001 eingereichte Anmeldung DE 10107002 des gleichen Anmelders beschreibt ein fermentatives Verfahren zur Herstellung von O-Acetyl-L-Serin. Damit ist zwar ein kostengünstiges Produktionssystem verfügbar, jedoch ist die Produktisolierung aus der Fermenterbrühe aufgrund der Instabilität des O-Acetyl-L-Serin problematisch.

Ein Vorzug der vorliegenden Erfindung ist es, dass eine O-Acetyl-L-Serin-haltige Fermenterbrühe wie sie z.B. aus einer nach DE 10107002 durchgeführten Fermentation erhalten wird, als O-Acetyl-L-Serin-Quelle direkt im erfindungsgemäßen Verfahren eingesetzt werden kann. Diese Vorgehensweise ist besonders ökonomisch und vermeidet die Isolierung einer instabilen Verbindung.

Die Herstellung von O-Acetyl-L-Serin-Sulfhydrylasen für die Synthese von nicht-proteinogenen Aminosäuren erfolgt vorzugsweise mit Hilfe von üblichen rekombinanten DNS-Techniken, wie sie dem Fachmann geläufig sind.

Ein Gen, das für eine O-Acetyl-L-Serin-Sulfhydrylase kodiert, wird hierfür in einen geeigneten Vektor kloniert und anschließend ein geeigneter Wirtsstamm transformiert. Als Wirtsstamm eignet sich jeder Mikroorganismus, der rekombinanten DNS-Techniken zugänglich ist und der für eine fermentative Herstellung von rekombinanten Proteinen geeignet ist.

Ein bevorzugter Mikroorganismus zur Herstellung von O-Acetyl-L-Serin-Sulfhydrylasen ist Escherichia coli.

Prinzipiell ist sowohl die chromosomale Integration des rekombinanten O-Acetyl-L-Serin-Sulfhydrylase-Gens als auch die Verwendung auf einem selbstreplizierenden Plasmid-Vektor möglich.

Bei der Klonierung werden bevorzugt Vektoren verwendet, die bereits genetische Elemente (z.B. regulierbare Promotoren, Terminatoren) enthalten, die eine kontrollierte, stark induzierbare Expression des O-Acetyl-L-Serin-Sulfhydrylase-Gens ermöglichen. Besonders bevorzugt sind Plasmid-Vektoren mit hoher Kopienzahl wie beispielsweise die Escherichia coli-Vektoren pUC18, pBR322, pACYC184 und ihre Derivate. Als stark induzierbare Promotoren eigenen sich beispielsweise lac-, tac-trc-, lambda P_{L}, ara- oder tet-Promotoren.

Die Produktion von O-Acetyl-L-Serin-Sulfhydrylasen erfolgt beispielsweise durch Kultivierung eines rekombinanten Mikroorganismenstammes durch Fermentation. Hierbei werden dem Fachmann bekannte Anzuchtverfahren angewendet, wobei die Verfahrensparameter dem jeweiligen Mikroorganismenstamm angepaßt sein müssen. Als Nährmedien können sowohl Vollmedien als auch Minimalmedien zum Einsatz kommen. Es kann sowohl ein batchals auch ein fed-batch-Prozeß angewendet werden. Bei Verwendung von induzierbaren Promotorsystemen wird zu einem geeigneten Zeitpunkt die Expression des O-Acetyl-L-Serin-Sulfhydrylase-Gens durch Zugabe eines entsprechenden Induktors angeschaltet. Nach einer ausreichenden Produktionsphase werden die O-Acetyl-L-Serin-Sulfhydrylase-haltigen Zellen durch bekannte Methoden (z.B. Zentrifugation) geerntet.

Eine Isolierung der O-Acetyl-L-Serin-Sulfhydrylase zur Herstellung von nicht-proteinogenen Aminosäuren ist nicht notwendig, die Mikroorganismenzellen, die O-Acetyl-L-Serin-Sulfhydrylase-Aktivität besitzen, werden direkt im erfindungsgemäßen Verfahren eingesetzt. Beispiele für solche Mikroorganismenstämme sind die Escherichia coli Stämme DH5α/pFL145 und BLR21(DE3)/pLE4. Sie werden in den Beispielen 1 und 2 beschrieben und wurden bei der Deutschen Sammlung für Mikroorganismen und Zellkulturen DSMZ in Braunschweig unter der Nr. DSM 14088 bzw. 14089 hinterlegt.

Somit erfolgt erfindungsgemäß eine Biotransformation von O-Acetyl-L-Serin zu einer nicht-proteinogenen L-Aminosäure mit ruhenden Zellen. Die Penetration der Zellen mit O-Acetyl-L-Serin und einer nukleophilen Verbindung ist dabei ebenso gegeben wie die Freisetzung des Reaktionsproduktes, der nicht-proteinogenen L-Aminosäure, durch die Zellen.

Falls erwünscht, kann der Stoffaustausch zwischen Zellinnerem und Reaktionsmedium erhöht werden, in dem die Zellen mit Substanzen behandelt werden, die eine Permeabilisierung der Zellen bewirken. Solche Substanzen, wie beispielsweise Chloroform oder Toluol, und deren Anwendung sind dem Fachmann bekannt.

In einer bevorzugten Ausführungsform des Verfahrens wird eine nicht-proteinogene Aminosäure durch Reaktion von O-Acetyl-L-Serin mit einer nukleophilen Verbindung unter Katalyse durch eine O-Acetyl-L-Serin-Sulfhydrylase gebildet, wobei die O-Acetyl-L-Serin-Sulfhydrylase intrazellulär in einem Mikroorganismus vorliegt.

Besonders bevorzugt ist die Herstellung von nicht-proteinogenen L-Aminosäuren durch Reaktion von fermentativ gewonnenem, nicht aufgereinigtem O-Acetyl-L-Serin mit einer nukleophilen Verbindung unter Katalyse durch eine O-Acetyl-L-Serin-Sulfhydrylase, wobei die O-Acetyl-L-Serin-Sulfhydrylase intrazellulär in einem Mikroorganismus vorliegt.

Als nukleophile Verbindungen für die durch O-Acetyl-Serin-Sulfhydrylasen katalysierte β-Substitution werden im erfindungsgemäßen Verfahren vorzugsweise Verbindungen eingesetzt, die einen Rest ausgewählt aus der Gruppe
-S-, -Se-, -N=N⁺=N⁻ -C≡N, 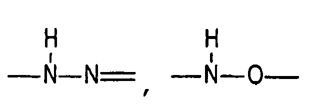 enthalten.

Besonders bevorzugt wird dem Reaktionsansatz eine nukleophile Verbindung ausgewählt aus der Gruppe der folgenden Verbindungen zugegeben:
- Thiosulfate
- Thiole der allgemeinen Formel (1):

   H-S-R¹ (1)
wobei R¹ einwertiger substituierter oder nicht substituierter Alkyl-, Alkoxy-, Aryl- oder Heteroarylrest mit 1 bis 15 C-Atomen bedeutet;
- Selenide
- Selenole der allgemeinen Formel (2)

   H-Se-R¹ (2)
wobei R¹ die zu Formel (1) genannte Bedeutung hat,
- Azide
- Cyanide
- Azole der allgemeinen Formel (3) oder (4): 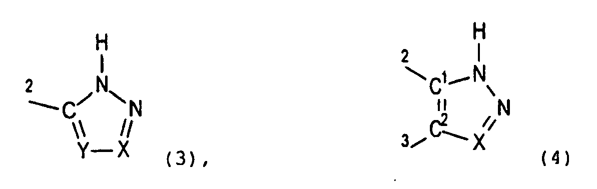
wobei X und Y gleich oder verschieden sind und CR⁴ oder N bedeuten und R⁴ -H, -COOH, -OH, -NH₂, -NO₂, -SH, -SO₃⁻, -F, -Cl,-Br, -I, C₁-C₅-Alkylcarbonyl- sowie deren Ester, Amide oder Salze oder R¹ bedeutet und R¹ die zu Formel (1) genannte Bedeutung hat und
wobei R² und R³ gleich oder verschieden sind und R⁴ bedeuten oder wobei C¹ und C² in Formel (4) anstelle der Substituenten R² und R³ mittels einer Brücke [-CR⁵R⁶-]ₐ mit a gleich 1, 2, 3 oder 4 zu einem Ring verknüpft sind, wobei
R⁵ und R⁶ gleich oder verschieden sind und R⁴ bedeuten und eine oder mehrere nicht benachbarte Gruppen [-CR⁵R⁶-] durch Sauerstoff, Schwefel oder einen ggf. mit C₁-C₅-Alkyl- substituierten Iminorest ersetzt sein können und
zwei benachbarte Gruppen [-CR⁵R⁶-] durch eine Gruppe [-CR⁵=CR⁶-] oder durch eine Gruppe [-CR⁵=N-] ersetzt sein können,
- Isoxazolinone der allgemeinen Formel (5) oder (6) : 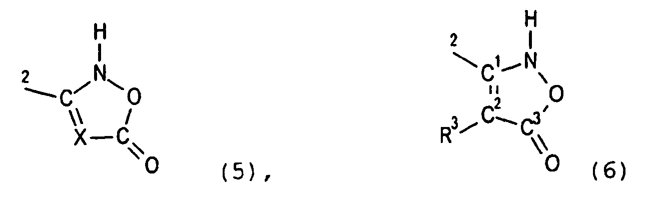
wobei X, R¹ , R² und R³ die bereits genannte Bedeutung haben und
wobei C¹ und C² in Formel (6) anstelle der Substituenten R² und R³ mittels einer wie für Formel (4) definierten Brücke zu einem Ring verknüpft sein kann.

Beispiele für Thiosulfate sind Natriumthiosulfat, Kaliumthiosulfat oder Ammoniumthiosulfat.

Beispiele für Thiole sind Verbindungen ausgewählt aus der Gruppe 2-Mercaptoethanol, 3-Mercaptopropanol, 3-Mercaptopropionsäure, 3-Mercapto-1-propansulfonsäure, Mercaptoethansulfonsäure, 2-Mercaptoethylamin, Thioglycolsäure, Thiomilchsäure, Thioessigsäure, Mercaptobernsteinsäure, Mercaptobrenztraubensäure, Dithiothreitol, Dithioerythritol, 1-Thioglycerin, Thiophenol, 4- Fluorthiophenol, 4-Chlorthiophenol, 4-Mercaptophenol, p-Thiokresol, 5-Thio-2-nitrobenzoesäure, 2-Mercaptothiazol, 2-Mercaptothiazolin, 2-Mercaptoimidazol, 3-Mercapto-1,2,4-Triazol, 2-Thiophenthiol, 2-Mercaptopyridin, 2-Mercaptopyrimidin, 2-Thiocytosin, 2-Mercaptonicotinsäure, 2-Mercapto-1-methylimidazol, 2-Mercaptobenzothiazol, 2-Mercaptobenzoxazol, 6-Mercaptopurin.

Bespiele für Selenole sind Verbindungen ausgewählt aus der Gruppe Methylselenol, Ethylselenol, Propylselenol, Phenylselenol.

Beispiele für Azide sind Natriumazid, Kaliumazid oder Ammoniumazid.

Beispiele für Cyanide sind Kaliumcyanid, Natriumcyanid oder Ammoniumcyanid.

Beispiele für Azole sind Verbindungen ausgewählt aus der Gruppe 1,2-Pyrazol, 3-Methylpyrazol, 4-Methylpyrazol, 3,5-Dimethylpyrazol, 3-Aminopyrazol, 4-Aminopyrazol, Pyrazol-4-carbonsäure, Pyrazol-3,5-dicarbonsäure, 1,2,3-Triazol, 1,2,4-Triazol, 3-Amino-1,2,4-Triazol, 1,2,3,4-Tetrazol, Indazol, Indazol-3-carbonsäure, Indazol-5-carbonsäure, 5-Aminoindazol, Benzotriazol, Benzotriazol-5-carbonsäure, 5-Aminobenzotriazol, Aminopyrazolopyrimidin, 8-Azaguanin, 8-Azaadenin.

Beispiele für Isoxazolinone sind Verbindungen ausgewählt aus der Gruppe Isoxazolin-5-on, 3-Methylisoxazolin-5-on, 4-Methylisoxazolin-5-on, 4,5-Dimethylisoxazolin-2-on, 1,2,4-Oxadiazolidin-3,5-dion.

Die Konzentration der nukleophilen Verbindung wird im Ansatz vorzugsweise so gewählt, dass sie in äquimolarer Konzentration zu O-Acetyl-L-Serin vorliegt.

Die Reaktionstemperatur wird vorzugsweise zwischen 5°C und 70°C gewählt. Der besonders bevorzugte Temperaturbereich liegt zwischen 20°C und 40°C.

Als Lösungsmittel für die Reaktion wird bevorzugt Wasser verwendet.

Als Reaktionsprodukte werden vorzugsweise L-Aminosäuren der allgemeinen Formel (7) in L-Konfiguration gebildet 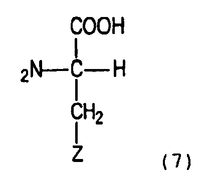 wobei Z ein einwertiger Rest ausgewählt aus den Formeln (8) bis (19)

-S-SO₃⁻ (8),

-S-R¹ (9),

-Se-H (10),

-Se-R¹, (11),

-N=N⁺=N⁻ (12),

-C≡N (13),

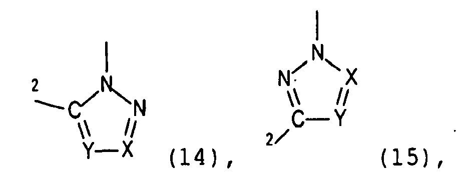

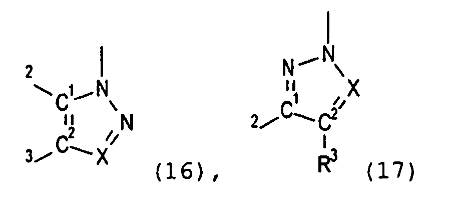

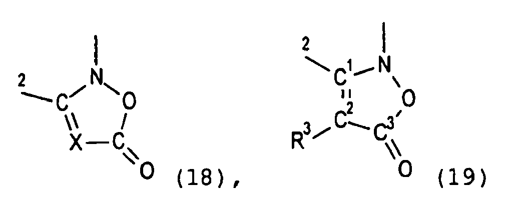

sowie deren Ester, Ether oder Salze ist,
und R¹, R², R³, R⁴, X und Y die bereits zu den Formeln (1) bis (6) genannte Bedeutung haben.

Vorzugsweise wird eine lösliche nicht-proteinogene L-Aminosäure nach Beendigung des erfindungsgemäßen Verfahrens und nach einer Trennung der vorzugsweise wässrigen Lösung mittels bekannter Methoden in Biomasse und einen Kulturüberstand aus dem Kulturüberstand isoliert. Solche Methoden zur Isolierung von Aminosäuren sind dem Fachmann ebenfalls bekannt. Sie umfassen z.B. Filtration, Zentrifugation, Extraktion, Adsorption, Ionenaustauscher-Chromatographie, Präzipitation, Kristallisation. Beim Vorliegen einer schwerlöslichen nicht-proteinogenen Aminosäure wird vorzugsweise eine klassierende Zentrifugation, wie sie dem Fachmann bekannt ist, durchgeführt, wobei die Biomasse möglichst im Zentrifugationsüberstand verbleibt. Das abgeschiedene Produkt wird vorzugsweise durch klassische Methoden gelöst und umgefällt.

Die folgenden Beispiele dienen der weiteren Erläuterung der Erfindung.

### Beispiel 1: Klonierung der cysK- bzw. cysM-Gene aus Escherichia coli als Fusionsgen mit StrepTag-Sequenz

Zur Klonierung der cysK- bzw. cysM-Gene aus Escherichia coli wurden zunächst Polymerase-Ketten-Reaktionen mit den folgenden phosphorothioat-geschützten Oligonukleotid-Primer-Paaren mit Hilfe der Pwo-DNA-Polymerase durchgeführt.
cysKS1: 5'-gat cga ggt ctc gaa tga gta aga ttt ttg aag a-3' SEQ.ID.NO. 1
cysKS2: 5'-gat cga ggt ctc ggc gct ctg ttg caa ttc ttt ctc ag-3' SEQ.ID.NO. 2 bzw.
cysMS3: 5'-gat cga ggt ctc gaa tga gta cat tag aac aaa c-3 SEQ.ID.NO.3
cysMS5: 5'-gat cga ggt ctc ggc gct aat ccc cgc ccc ctg gct aa-3' SEQ.ID.NO. 4
Dabei wurden über die Primer-Sequenzen Schnittstellen für die Restriktionsendonuklease BsaI (unterstrichene Nukleotide) eingeführt. Als Matritze diente chromosomale DNA von E. coli W3110 (ATTC 27325). Die erhaltenen Amplifikate wurden mit dem Restriktionsenyzm BsaI verdaut und anschließend in den mit Eco31I-behandelten Vektor pASK-IBA3 (Institut für Bioanalytik, Göttingen, D) kloniert. Dieser Vektor erlaubt eine Klonierung als Genfusion mit einer Sequenz am 3'-Ende, die für ein Affinitätspeptid (WSHPQFEK SEQ.ID.NO. 5) mit der Bezeichnung StrepTagII kodiert. Die Expression der Genfusion mit Hilfe des tet-Promoters führt zu einem Protein mit C-terminalem StrepTagII-Anhängsel. Letzteres kann zur Isolierung des Proteins benützt werden, da das StrepTagII eine Bindung an Streptavidin-Säulen vermittelt.

### Beispiel 2: Reinigung der CysK- bzw. CysM-Proteine

Mit dem cysM-StrepTag-Konstrukt (pFL145) konnte im E. coli Stamm DH5α (Clonetech, Heidelberg, D) eine sehr gute Genexpression verzeichnet werden. Dabei wurde die Anzucht und Expression nach den Angaben der Firma Institut für Bioanalytik, Göttingen, D, durchgeführt. Der Stamm DH5α/pFL145 wurde bei der Deutschen Sammlung für Mikroorganismen und Zellkulturen DSMZ gemäß Budapester Vertrag unter der Nummer DSMZ 14088 hinterlegt. Im Falle des cysK-StrepTag-Konstruktes (pLE1) war dagegen die Expression nur sehr schwach. Durch Umklonieren der Genfusion als XbaI-HindIII-Fragment in einen Vektor mit T7-Promotor (pRSETSa) (Stratagene, Heidelberg, D) und Expression im Stamm BLR21(DE3) (Novagen, Darmstadt, D) konnte jedoch auch in diesem Fall (pLE4) eine gute Bildung des Genproduktes erzielt werden. Der Stamm BLR21(DE3)/pLE4 wurde bei der Deutschen Sammlung für Mikroorganismen und Zellkulturen DSMZ gemäß Budapester Vertrag unter der Nummer DSMZ 14089 hinterlegt.
Die Isolierung der CysK- bzw. CysM-Proteine erfolgte durch Affinitätschromatographie an Streptavidinsäulen wobei prinzipiell nach den Herstellerangaben der Firma Institut für Bioanalytik, Göttingen,D, verfahren wurde. Die Ausbeute betrug bei Verwendung von 250 ml Kultur 3 mg gereinigtes CysK-Protein bzw. 4,5 mg gereinigtes CysM-Protein. Beide Proteine wiesen aufgrund des Pyridoxalphosphat-Cofaktors eine deutliche Gelbfärbung auf und besaßen ein Absorptionsmaximum bei 420 nm.

### Beispiel 3: Bestimmung der Enzymaktivität

Die Messung der Aktivität von O-Acetyl-L-Serin-Sulfhydrylasen erfolgt durch Nachweis des "natürlichen" Reaktionsproduktes L-Cystein (Cys) oder bei CysM alternativ auch als S-Sulfo-L-cystein (S-Cys). Beide Produkte lassen sich sehr spezifisch und sehr sensitiv mit dem Test nach Gaitonde (1967, Biochem. J. 104: 627-633) nachweisen. Für beide Verbindungen mussten separate Eichkurven aufgenommen werden, da der Farbkomplex mit Sulfocystein eine geringere Intensität aufweist.
Ein typischer Testansatz enthielt 10 mM O-Acetyl-Serin (Zugabe aus einer 200 mM Stammlösung in 500 mM Natrium-Succinat-Puffer pH 5,5), 10 mM Natriumsulfid oder Natriumthiosulfat, 100 mM Kalium-Phosphat-Puffer pH 7,0, 5 µg/ml gereinigtes Enyzm und wird bei 37 °C inkubiert. Die spezifische Aktivität A wird in Units / mg Protein angegeben d.h. in µmol Produkt x min⁻¹ x mg⁻¹ Protein. A_{Cys} bedeutet spezifische Aktivität der Cystein-Bildung mit Sulfid. A_{S-Cys} bedeutet spezifische Aktivität der S-Sulfo-cystein-Bildung mit Thiosulfat. Die spezifische Aktivität mit den in Beispiel 2 isolierten Proteinen betrug:
CysK: A_{Cys} 140 Units / mg
CysM: A_{Cys} 199 Units / mg
CysM: A_{S-Cys} 145 Units / mg

Bei der Bestimmung der Aktivität von O-Acetyl-Serin-Sulfhydrylasen in ruhenden Zellen wurde zunächst eine Zellsuspension mit einer optischen Dichte von 20,0 (gemessen bei 600 nm) eingestellt. Anschließend wurden die Zellen durch Zugabe von Chloroform ad 10 Vol % permeabilisiert und 5 min bei Raumtemperatur inkubiert. Für den Enzymtest wurde dann eine optische Dichte von 1,0 eingestellt und mit den Zellen wie oben beschrieben die Reaktion mit O-Acetyl-L-Serin und Sulfid bzw. Thiosulfat durchgeführt. Die spezifische Aktivität der Zellen
A wird hierbei angegeben in µmol x min⁻¹ x (ml Zellsuspension mit optischer Dichte von 1,0 bei 600 nm)⁻¹; in abgekürzter Form: Units / ml OD.

### Beispiel 4: Untersuchung der Katalysemöglichkeiten der O-Acetyl-L-Serin-Sulfhydrylasen CysK bzw. CysM

Um mit Hilfe der O-Acetyl-L-Serin-Sulfhydrylasen nichtproteinogene Aminosäuren herzustellen, wurden andere nukleophile Verbindungen anstelle von Sulfid oder Thiosulfat in die Reaktion eingesetzt. Zu deren Nachweis wurde eine Vorsäulenderivatisierung für Aminosäuren mit ortho-Phthaldialdehyd durchgeführt. Mit dieser Methode konnte ein breites Screening aufgeeignete nukleophile Substrate durchgeführt werden.

Die Ansätze enthielten 4 mM O-Acetyl-L-Serin (Zugabe aus einer 200 mM Stammlösung in 500 mM NaSuccinat-Puffer pH 5,5), 20 mM Nukleophil, 100 mM K-Phosphat-Puffer pH 7,0, 5 µg/ml gereinigtes Enyzm. Die Reaktion wurde mit 1/100 Volumen Essigsäure (96 v/v) abgestoppt und durch HPLC mit einer HP Aminoquant Säule (200 mm x 2,1 mm) nach Herstellerangaben von Hewlett Packard, Waldbronn, D, analysiert.

Um die gebildeten Produkte zu charakterisieren, wurde die Molekülmasse mittels HPLC-MS nachgewiesen. Hierfür diente eine Luna-C18-Säule (Phenomenex, Aschaffenburg, D) mit Ameisensäure (0,1 % v/v) als mobile Phase. Die Ionisierung wurde im positiven Elektrospray-Modus durchgeführt.

Die folgende Tabelle zeigt die Reaktivität der beiden Enzyme CysK bzw. CysM gegenüber verschiedenen Nukleophilen und die bestimmte Masse in der HPLC-MS-Analyse (MH+).

| **Nukleophil (bzw. Salz)** | **CysK *** | **CysM *** | **Produkt** | **MH+** |
|---|---|---|---|---|
| Mercaptoethanol | +++ | +++ | S-Hydroxyethyl-L-cystein | 166 |
| Dithiothreithol | +++ | +++ | S-Mercaptodihydroxybutyl-L-cystein | 242 |
| Thiophenol | ++ | +++ | S-Phenyl-L-cystein | 198 |
| 2-Mercaptothiazol | ++ | +++ | Thiazol-2-yl-L-cystein | 205 |
| 3-Mercapto-1,2,4-triazol | + | +++ | 1,2,4-Triazol-3-yl-L-cystein | 189 |
| Natriumselenid | +++ | +++ | L-Selenocystein | 170 |
| Phenylselenol | ++ | +++ | Phenyl-L-selenocystein | 246 |
| Natriumazid | +++ | +++ | Azido-L-alanin | 131 |
| Kaliumcyanid | +++ | +++ | Cyano-L-alanin | 115 |
| 1,2-Pyrazol | ++ | ++ | 1,2-Pyrazolyl-L-alanin | 156 |
| 1,2,4-Triazol | +++ | ++ | 1,2,4-Triazolyl-1-L-alanin | 157 |
| 1,2,3,4-Tetrazol | +++ | ++ | 1,2,3,4-Tetrazol-2-yl-L-alanin | 158 |
| 1,2,3-Benzotriazol | ++ | + | 1,2,3-Benzotriazol-2-yl-alanin | 207 |
| 5-Carboxy-1,2,3-Benzotriazol | ++ | + | 5-Carboxy-1,2,3-Benzotriazol-2-yl-alanin | 242 |
| 8-Azaguanin | - | + | Azaguan-8-yl-L-alanin | 240 |
| 1,2,9-Oxadiazolidin-3,5-dion | - | + | 1,2,4-Oxadiazolidindionyl-L-alanin | 190 |

| | | | | |
|---|---|---|---|---|
| * +++ Umsatz größer 70 % nach 30 min, ++ Umsatz größer 40 % nach 30 min, + Umsatz größer 10 % nach 30 min, - Umsatz kleiner 5 % nach 30 min. | | | | |

### Beispiel 5: Fermentative Herstellung der O-Acetyl-L-Serin-Sulfhydrylase CysM

Als Vorkultur für die Fermentation wurden 20 ml LB-Medium (10 g/l Trypton, 5 g/l Hefeextrakt, 10 g/l NaCl), das zusätzlich 100 mg/l Ampicillin enthielt, mit dem Stamm DH5α/pFL145 (s.o.) beimpft und bei 30 °C und 150 rpm in einem Schüttler über Nacht inkubiert. Anschließend wurde der gesamte Ansatz in 100 ml SM1-Medium (12 g/l K₂HPO₄; 3 g/l KH₂PO₄; 5 g/l (NH₄)₂SO₄; 0,3 g/l MgSO₄ x 7 H₂O; 0,015 g/l CaCl₂ x 2 H₂O; 0,002 g/l FeSO₄ x 7 H₂O; 1 g/l Na₃Citrat x 2 H₂O; 0,1 g/l NaCl; 1 ml/l Spurenelementlösung bestehend aus 0,15 g/l Na₂MoO₄ x 2 H₂O; 2,5 g/l Na₃BO₃; 0,7 g/l CoCl₂ x 6 H₂O; 0,25 g/l CuSO₄ x 5 H₂O; 1,6 g/l MnCl₂ x 4 H₂O; 0,3 g/l ZnSO₄ x 7 H₂O), das mit 5 g/l Glukose; 0,5 mg/l Vitamin B₁ und 100 mg/l Ampicillin supplementiert wurde, überführt. Die weitere Inkubation der Vorkultur erfolgte bei 30 °C für 8 Stunden bei 150 rpm.

Als Fermenter diente ein Biostat M-Gerät der Firma Braun Biotech (Melsungen, D) mit einem maximalen Kulturvolumen von 2 l. Mit der beschriebenen Vorkultur (optische Dichte bei 600 nm von ca. 2) wurde der Fermenter mit 900 ml Fermentationsmedium (15 g/l Glukose; 10 g/l Trypton; 5 g/l Hefeextrakt; 5 g/l (NH₄)₂SO₄; 1,5 g/l KH₂PO₄; 0,5 g/l NaCl; 0,3 g/l MgSO₄ x 7 H₂O; 0,015 g/l CaCl₂ x 2 H₂O; 0,075 g/l FeSO₄ x 7 H₂O; 1 g/l Na₃Citrat x 2 H₂O und 1 ml/l Spurenelementlösung s.o., 5 mg/l Vitamin B1 und 100 mg/l Ampicillin, eingestellt auf pH 7,0 mit 25 %-igem Ammoniak) beimpft. Während der Fermentation wurde eine Temperatur von 32 °C eingestellt und der pH-Wert durch Zudosierung von 25 % v/v Ammoniak bei einem Wert von 7,0 konstant gehalten. Die Kultur wurde mit entkeimter Druckluft bei 1,5 vol/vol/min begast und mit einer Rührerdrehzahl von 200 rpm gerührt. Nach Absinken der Sauerstoffsättigung auf einen Wert von 50 % wurde die Drehzahl über ein Kontrollgerät bis zu einem Wert von 1200 rpm erhöht, um 50 % Sauerstoffsättigung zu erhalten (Bestimmt mit einer pO₂-Sonde, kalibriert auf 100% Sättigung bei 900 rpm). Sobald der Glukose-Gehalt im Fermenter von anfänglich 15 g/l auf ca. 5-10 g/l abgesunken war, erfolgte eine Zudosierung einer 56 % w/v Glukose-Lösung. Die Fütterung erfolgte mit einer Flußrate von 3-6 ml/h, wobei die Glukosekonzentration im Fermenter zwischen 0,5 - 10 g/l gehalten wurde. Die Glukose-Bestimmung wurde mit dem Glukoseanalysator der Firma YSI (Yellow Springs, Ohio, USA) durchgeführt. Beim Erreichen einer optischen Dichte von 30 wurde die Enzymproduktion durch die Zugaben von 3 mg /l Tetracyclin induziert. Die Induktionsdauer betrug 7 Stunden. Nach dieser Zeit wurden Proben entnommen und die Zellen durch Zentrifugation vom Kulturmedium abgetrennt und gewaschen. Die resultierende Zellsuspension wurde wie in Beispiel 3 beschrieben analysiert. Die O-Acetyl-Serin-Sulfhydrylase-Aktivität gemessen mit Thiosulfat A_{S-Cys} betrug 12 Units / ml OD.
Die Zellen wurden durch Zentrifugation geerntet, in flüssigem Stickstoff schockgefroren und bei -20°C aufbewahrt.

### Beispiel 6: Fermentative Herstellung der O-Acetyl-L-Serin-Sulfhydrylase CysK

Für die Herstellung des CysK-Enyzms wurde prinzipiell vorgegangen wie in Beispiel 5 beschrieben. Allerdings wurde hier der Stamm BLR21(DE3)/pLE4 verwendet. Nach Verbrauch der vorgelegten 15 g/l Glukose wurde eine Fütterung mit einer 60 %-iger (v/v) Glycerinlösung aufgenommen. Die Flußrate betrug 9,5 ml/h. Gleichzeitig wurde die Enzymproduktion durch Zugabe von 0,4 mM Isopropyl-β-thiogalaktosid (IPTG) induziert. Nach einer Induktionszeit von 21 Stunden wurden Proben entnommen und die Zellen durch Zentrifugation vom Kulturmedium abgetrennt und gewaschen. Die resultierende Zellsuspension wurde wie in Beispiel 3 beschrieben analysiert. Die spezifische O-Acetyl-Serin-Sulfhydrylase-Aktivität gemessen mit Sulfid A_{Cys} betrug 2 Units / ml OD.
Die Zellen wurden durch Zentrifugation geerntet, in flüssigem Stickstoff schockgefroren und bei -20°C aufbewahrt.

### Beispiel 7: Enzymatische Herstellung von S-Phenyl-L-cystein mit ruhenden Zellen

In einem temperierbaren Gefäß mit pH-Sonde und pH-Steuerung wurde zunächst O-Acetyl-L-Serin-Hydrochlorid (Sigma, Deisenhofen, D) in 100 ml Wasser gelöst, so dass eine Endkonzentration von 200 mM (29,4 g/l) O-Acetyl-L-Serin vorlag. Danach wurde unter Rührung 200 mM Thiophenol zugegeben. Anschließend wurde der Ansatz durch Titration mit 5 M NaOH zügig auf einen pH-Wert von 6,7 gebracht und sofort eine Zellsuspension des CysM-Produktionsstammes DH5α/pFL145 eingemischt. Die Zellsuspension besaß eine spezifische Aktivität A_{S-Cys} von 12 Units / ml OD. Die optische Dichte (gemessen bei 600 nm) der Zellen im Ansatz betrug 2,0 und damit die O-Acetyl-Serin-Sulfhydrylase-Aktivität einen Wert A_{S-Cys} von 24 Units / ml. Während der Reaktion wurde der pH-Wert durch Dosierung von 5 M NaOH über das pH-Kontrollsystem konstant gehalten.
Während der Reaktion wurde die Bildung eines weißen Präzipitates beobachtet. Nach 30 min wurde eine Probe entnommen und mit 1 %-iger (v/v) Essigsäure versetzt. Der Niederschlag wurde durch Zentrifugation abgetrennt, in gleichem Volumen 21 %-iger (v/v) Phosphorsäure gelöst und durch HPLC analysiert. Der Überstand der Zentrifugation wurde ebenfalls chromatographiert. Die Analyse wurde mittels reversed phase HPLC an einer LUNA 5 µ C18(2)-Säule (Phenomenex, Aschaffenburg, D) durchgeführt. Als Eluent diente verdünnte Phosphorsäure (0,1 ml konz. Phosphorsäure / l) bei einer Flußrate von 0,5 ml/min.

Der Gehalt an S-Phenyl-L-Cystein betrug im Überstand 1,6 g/l und im Niederschlag 24,8 g/l. Dies entspricht einer Gesamtreaktionsausbeute von 67 % bezogen auf O-Acetyl-L-Serin. Der Ansatz wurde auch mit reduziertem Einsatz an O-Acetyl-Serin-Sulfhydrylase auf 12 bzw. 2 Units / ml durchgeführt. Dies führte jedoch zu einer verlangsamten Enyzmreaktion und aufgrund der Isomerisierung von O-Acetyl-L-Serin zu verminderten Ausbeuten von 52 bzw. 15 %.

### Beispiel 8: Enzymatische Herstellung von S-Phenyl-L-cystein mit O-Acetyl-L-Serin-haltiger Fermenterbrühe und ruhenden Zellen.

Anstatt O-Acetyl-L-Serin als Reinsubstanz zu verwenden, wurde dieser Versuch in O-Acetyl-L-Serin-haltiger Fermenterbrühe durchgeführt. Diese wurde durch eine Fermentation, wie in der Patentanmeldung DE 10107002 beschrieben, gewonnen. Am Ende der Fermentation wurde zur Stabilisierung von O-Acetyl-L-Serin ein pH-Wert von 4,0 mit 21 %-iger (v/v) Phosphorsäure eingestellt. Die Zellen wurden durch Zentrifugation abgetrennt und die Fermenterbrühe durch Eindampfen auf eine Konzentration von 30 g/l O-Acetyl-L-Serin gebracht. Anschließend wurden zu 100 ml Brühe 200 mM Thiophenol und nach Titration auf pH 6,7 Zellen des CysM-Produktionsstammes DH5α/pFL145 eingemischt. Die Aktivität A_{S-CYS} betrug 24 Units / ml. Danach wurde wie in Beispiel 7 weiterverfahren. Die Ausbeute bezogen auf O-Acetyl-L-Serin betrug 65 %.
Ein identischer Ansatz mit Zellen des CysK-Produktionstammes BLR21(DE3)/pLE4 lieferte bei Einsatz von A_{Cys} 38 Units / ml eine Ausbeute von 72 %.

### Beispiel 9: Kontinuierliche enzymatische Herstellung von S-Phenyl-L-Cystein mit O-Acetyl-L-Serin-haltiger Fermenterbrühe und ruhenden Zellen.

Ein Ziel der Reaktionsführung ist es, möglichst hohe Umsätze bezogen auf das teure und instabile Edukt O-Acetyl-L-Serin zu erreichen. Um dies zu gewährleisten wurde ein kontinuierliches Verfahren ausgearbeitet. Dabei wird zunächst vorgegangen wie in Beispiel 8 beschrieben, lediglich der Einsatz an CysM-Aktivität A_{S-Cys} wurde auf 48 Units / ml erhöht. Nach 20 min Reaktionszeit wurde über Dosierungseinheiten O-Acetyl-L-Serin-haltige Fermenterbrühe (30 g/l), Thiophenol und eine Zellsuspension des CysM-Produktionsstammes DH5α/pFL145 (240 OD bei 600 nm, in Kalium-Phosphatpuffer pH 7,0) zugegeben. Die Flußraten betrugen 150, 2,5, bzw. 3 ml/h. Gleichzeitig wurde damit gestartet, kontinuierlich Produktlösung aus dem Reaktionsgefäß mit einer Flußrate von 155,5 ml/h zu entnehmen. Die Flußraten wurden so gewählt, dass das Reaktionsvolumen im Reaktor konstant blieb und die mittlere Verweilzeit im Reaktor 30 min betrug. Mit diesem Vorgehen stellte sich im Ansatz ein Fließgleichgewicht mit einer konstanten O-Acetyl-L-Serin-Konzentration von 1,0 g/l ein. Aufgrund dieser niedrigen Konzentration - bei der das Enzym aufgrund seines noch niedrigeren KM-Wertes noch sehr gute Aktivität besitzt - konnte die Isomerisierung zu N-Acetyl-L-Serin erfolgreich minimiert werden. Die Reaktionsausbeute bezogen auf O-Acetyl-L-Serin betrug 85 %.

### Beispiel 10: Enzymatische Herstellung von Azido-L-Alanin mit O-Acetyl-L-Serin-haltiger Fermenterbrühe und ruhenden Zellen.

In diesem Beispiel wurde prinzipiell wie in Beispiel 8 mit CysM-haltigen Zellen verfahren. Anstelle von Thiophenol wurden 200 mM Natriumazid eingesetzt. Nach 30 min Reaktionszeit mit Zellen entsprechend einer Aktivität von 72 Units / ml im Ansatz wurde ein Umsatz von 45 % bestimmt.

### Beispiel 11: Enzymatische Herstellung von Cyano-L-Alanin mit O-Acetyl-L-Serin-haltiger Fermenterbrühe und ruhenden Zellen.

In diesem Beispiel wurde prinzipiell wie in Beispiel 8 mit CysM-haltigen Zellen verfahren. Anstelle von Thiophenol wurden 200 mM Kaliumcyanid eingesetzt. Die Zugabe des Cyanids erfolgte erst nachdem der Ansatz auf einen pH-Wert von 6,7 eingestellt wurde. Nach 30 min Reaktionszeit mit Zellen entsprechend einer Aktivität von 72 Units/ml im Ansatz wurde ein Umsatz von 65 % bestimmt.

### Beispiel 12: Enzymatische Herstellung von S-Sulfo-L-Cystein mit O-Acetyl-L-Serin-haltiger Fermenterbrühe und ruhenden Zellen.

In diesem Beispiel wurde prinzipiell wie in Beispiel 8 mit CysM-haltigen Zellen verfahren. Anstelle von Thiophenol wurden 200 mM Natriumthiosulfat eingesetzt. Nach 30 min Reaktionszeit mit Zellen entsprechend einer Aktivität von 16 Units/ml im Ansatz wurde ein Umsatz von 91 % bestimmt. **Beispiel 13: Enzymatische Herstellung von S-Thiazol-2-yl-L-Cystein mit O-Acetyl-L-Serin-haltiger Fermenterbrühe und ruhenden Zellen.**

In diesem Beispiel wurde prinzipiell wie in Beispiel 8 mit CysM-haltigen Zellen verfahren. Anstelle von Thiophenol wurden 200 mM 2-Mercaptothiazol eingesetzt. Nach 30 min Reaktionszeit mit Zellen entsprechend einer Aktivität von 72 Units/ml im Ansatz wurde ein Umsatz von 62 % bestimmt.

### Beispiel 14: Enzymatische Herstellung von S-1,2,4-Triazol-3-yl-L-cystein mit O-Acetyl-L-Serin-haltiger Fermenterbrühe und ruhenden Zellen.

In diesem Beispiel wurde prinzipiell wie in Beispiel 8 mit CysM-haltigen Zellen verfahren. Anstelle von Thiophenol wurden 200 mM 3-Mercapto-1,2,4-triazol eingesetzt. Nach 30 min Reaktionszeit mit Zellen entsprechend einer Aktivität von 72 Units/ml im Ansatz wurde ein Umsatz von 74 % bestimmt.

### Beispiel 15: Enzymatische Herstellung von Seleno-L-Cystein mit O-Acetyl-L-Serin-haltiger Fermenterbrühe und ruhenden Zellen.

In diesem Beispiel wurde prinzipiell wie in Beispiel 8 mit CysM-haltigen Zellen verfahren. Anstelle von Thiophenol wurden 200 mM Natriumselenid eingesetzt. Das Natriumselenid wurde durch Reduktion von Natriumselenit mit Natriumborhydrid hergestellt. Nach 30 min Reaktionszeit mit Zellen entsprechend einer Aktivität von 72 Units/ml im Ansatz wurde ein Umsatz von 75 % bestimmt.

### Beispiel 16: Enzymatische Herstellung von Phenyl-seleno-L-Cystein mit O-Acetyl-L-Serin-haltiger Fermenterbrühe und ruhenden Zellen.

In diesem Beispiel wurde prinzipiell wie in Beispiel 8 mit CysM-haltigen Zellen verfahren. Anstelle von Thiophenol wurden 200 mM Phenylselenol eingesetzt. Nach 30 min Reaktionszeit mit Zellen entsprechend einer Aktivität von 72 Units/ml im Ansatz wurde ein Umsatz von 62 % bestimmt.

### Beispiel 17: Enzymatische Herstellung von 1,2,4-Triazol-1-yl-L-Alanin mit O-Acetyl-L-Serin-haltiger Fermenterbrühe und ruhenden Zellen.

In diesem Beispiel wurde prinzipiell wie in Beispiel 8 verfahren. Anstelle von Thiophenol wurden 200 mM 1,2,4-Triazol eingesetzt. Für diese Reaktion wurden die Zellen durch eine Behandlung mit 10 % (v/v) Chloroform permeabilisiert. Nach 30 min Reaktionszeit mit Zellen entsprechend einer Aktivität von 72 Units/ml im Ansatz wurde ein Umsatz von 48 % bestimmt.

### Beispiel 18: Enzymatische Herstellung von 5-Carboxy-1,2,3-Benzotriazol-2-yl-L-Alanin mit O-Acetyl-L-Serin-haltiger Fermenterbrühe und ruhenden Zellen.

In diesem Beispiel wurde prinzipiell wie in Beispiel 8 verfahren. Anstelle von Thiophenol wurden 200 mM 5-Carboxy-1,2,3-Benzotriazol eingesetzt. Für diese Reaktion wurden die Zellen durch eine Behandlung mit 10 % Chloroform permeabilisiert.
Nach 30 min Reaktionszeit mit Zellen entsprechend einer Aktivität von 72 Units/ml im Ansatz wurde ein Umsatz von 54 % bestimmt.

### Beispiel 19: Enzymatische Herstellung von 1,2,4-Oxadiazolidin-3,5-dionyl-L-alanin (=Quisqualsäure) mit O-Acetyl-L-Serin-haltiger Fermenterbrühe und ruhenden Zellen.

In diesem Beispiel wurde prinzipiell wie in Beispiel 8 verfahren. Anstelle von Thiophenol wurden 200 mM 1,2,4-Oxadiazolidin-2,5-dion eingesetzt. Für diese Reaktion wurden die Zellen durch eine Behandlung mit 10 % Chloroform permeabilisiert. Nach 30 min Reaktionszeit mit Zellen entsprechend einer Aktivität von 72 Units/ml im Ansatz wurde ein Umsatz von 11 % bestimmt.

## Patentansprüche

1. Verfahren zur Herstellung einer nicht-proteinogenen L-Aminosäure mittels einer enzymatischen Biotransformation, bei dem O-Acetyl-L-Serin mit einer nukleophilen Verbindung unter Katalyse einer O-Acetyl-L-Serin-Sulfhydrylase zu einer nicht-proteinogenen L-Aminosäure umgesetzt wird, **dadurch gekennzeichnet, daß** die Biotransformation unter Einsatz von ruhenden Mikroorganismenzellen, die eine O-Acetyl-L-Serin-Sulfhydrylase Aktivität besitzen, erfolgt und das Verfahren bei einem pH-Wert im Bereich zwischen pH 6,0 und 6,99 durchgeführt wird.

2. Verfahren gemäß Anspruch 1, **dadurch gekennzeichnet, daß** als O-Acetyl-L-Serin-Sulfhydrylase CysM eingesetzt wird.

3. Verfahren gemäß Anspruch 1 oder 2, **dadurch gekennzeichnet, daß** die O-Acetyl-Serin-Sulfhydrylase eine Volumenaktivität A_{Cys} von mindestens 2 Units / ml im Ansatz besitzt.

4. Verfahren gemäß einem der Ansprüche 1 bis 3, **dadurch gekennzeichnet, daß** als O-Acetyl-L-Serin eine O-Acetyl-L-Serin-haltige Fermenterbrühe eingesetzt wird.

5. Verfahren gemäß einem der Ansprüche 1 bis 4, **dadurch gekennzeichnet, daß** die nukleophile Verbindung einen Rest ausgewählt aus der Gruppe
H-S-, H-Se-, -N=N⁺=N⁻, -C≡N, 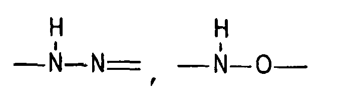 enthält.

6. Verfahren gemäß Anspruch 5, **dadurch gekennzeichnet, daß** die nukleophile Verbindung ausgewählt ist aus der Gruppe der Thiosulfate, der Thiole der allgemeinen Formel (1) :
H-S-R¹ (1)
wobei R¹ einwertiger substituierter oder nicht substituierter Alkyl-, Alkoxy-, Aryl- oder Heteroarylrest mit 1 bis 15 C-Atomen bedeutet,
der Selenide, der Selenole der allgemeinen Formel (2)
H-Se-R¹ (2)
wobei R¹ die zu Formel (1) genannte Bedeutung hat,
der Azide, der Cyanide, der Azole der allgemeinen Formel (3) oder (4): 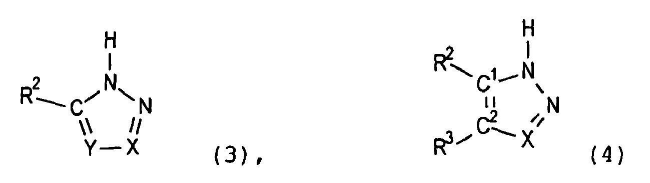 wobei X und Y gleich oder verschieden sind und CR⁴ oder N bedeuten und R⁴ -H, -COOH, -OH, -NH₂, -NO₂⁻, -SH, -SO₃⁻, -F, -Cl, -Br, -I, C₁-C₅-Alkylcarbonyl- sowie deren Ester, Amide oder Salze oder R¹ bedeutet und R¹ die zu Formel (1) genannte Bedeutung hat und
wobei R² und R³ gleich oder verschieden sind und R⁴ bedeuten oder wobei C¹ und C² in Formel (4) anstelle der Substituenten R² und R³ mittels einer Brücke [-CR⁵R⁶-]ₐ mit a gleich 1, 2, 3 oder 4 zu einem Ring verknüpft sind, wobei
R⁵ und R⁶ gleich oder verschieden sind und R⁴ bedeuten und eine oder mehrere nicht benachbarte Gruppen [-CR⁵R⁶-] durch Sauerstoff, Schwefel oder einen ggf. mit C₁-C₅-Alkyl- substituierten Iminorest ersetzt sein können und
zwei benachbarte Gruppen [-CR⁵R⁶-] durch eine Gruppe [-CR⁵=CR⁶-] oder durch eine Gruppe [-CR⁵=N-] ersetzt sein können,
der Isoxazolinone der allgemeinen Formel (5) oder (6): 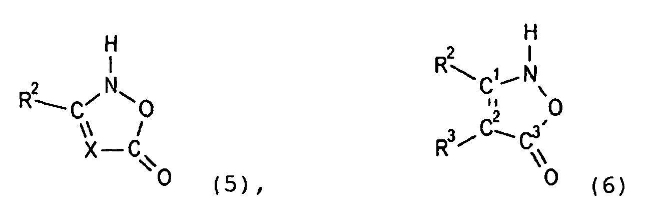 wobei X, R¹ , R² und R³ die bereits genannte Bedeutung haben und
wobei C¹ und C² in Formel (6) anstelle der Substituenten R² und R³ mittels einer wie für Formel (4) definierten Brücke zu einem Ring verknüpft sein kann.

7. Verfahren gemäß Anspruch 5 oder 6, **dadurch gekennzeichnet, daß** die nukleophile Verbindung ausgewählt ist aus der Gruppe 2-Mercaptoethanol, 3-Mercaptopropanol, 3-Mercaptopropionsäure, 3-Mercapto-1-propansulfonsäure, Mercaptoethansulfonsäure, 2-Mercaptoethylamin, Thioglycolsäure, Thiomilchsäure, Thioessigsäure, Mercaptobernsteinsäure, Mercaptobrenztraubensäure, Dithiothreitol, Dithioerythritol, 1-Thioglycerin, Thiophenol, 4- Fluorthiophenol, 4-Chlorthiophenol, 4-Mercaptophenol, p-Thiokresol, 5-Thio-2-nitrobenzoesäure, 2-Mercaptothiazol, 2-Mercaptothiazolin, 2-Mercaptoimidazol, 3-Mercapto-1,2,4-Triazol, 2-Thiophenthiol, 2-Mercaptopyridin, 2-Mercaptopyrimidin, 2-Thiocytosin, 2-Mercaptonicotinsäure,2-Mercapto-1-methylimidazol, 2-Mercaptobenzothiazol, 2-Mercaptobenzoxazol, 6-Mercaptopurin, Natriumthiosulfat, Kaliumthiosulfat, Ammoniumthiosulfat, Natriumazid, Kaliumazid, Ammoniumazid, Kaliumcyanid, Natriumcyanid, Ammoniumcyanid, Methylselenol, Ethylselenol, Propylselenol, Phenylselenol, 1,2-Pyrazol, 3-Methylpyrazol, 4-Methylpyrazol, 3,5-Dimethylpyrazol, 3-Aminopyrazol, 4-Aminopyrazol, Pyrazol-4-carbonsäure, Pyrazol-3,5-dicarbonsäure, 1,2,3-Triazol, 1,2,4-Triazol, 3-Amino-1,2,4-Triazol, 1,2,3,4-Tetrazol, Indazol, Indazol-3-carbonsäure, Indazol-5-carbonsäure, 5-Aminoindazol, Benzotriazol, Benzotriazol-5-carbonsäure, 5-Aminobenzotriazol, Aminopyrazolopyrimidin, 8-Azaguanin, 8-Azaadenin, Isoxazolin-5-on, 3-Methylisoxazolin-5-on, 4-Methylisoxazolin-5-on, 4,5-Dimethylisoxazolin-2-on, 1,2,4-Oxadiazolidin-3,5-dion.

8. Verfahren gemäß einem der Ansprüche 1 bis 7, **dadurch gekennzeichnet, daß** die Konzentration der nukleophilen Verbindung so gewählt ist, dass sie in einer äquimolaren Konzentration zu O-Acetyl-L-Serin vorliegt.

9. Verfahren gemäß einem der Ansprüche 1 bis 8, **dadurch gekennzeichnet, daß** es bei einer Temperatur zwischen 5°C und 70°C

10. Verfahren gemäß einem der Ansprüche 1 bis 9, **dadurch gekennzeichnet, daß** die unnatürliche L-Aminosäure ausgewählt ist aus der Gruppe der Aminosäuren der allgemeinen Formel (7) in L-Konfiguration 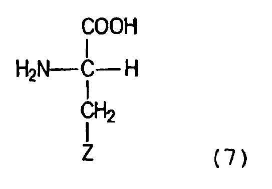 wobei Z ein einwertiger Rest gemäß einer der Formeln (8) bis (19)
-S-SO₃⁻ (8),
-S-R¹ (9),
-Se-H (10),
-Se-R¹ (11),
-N=N⁺=N⁻ (12),
-C≡N (13), 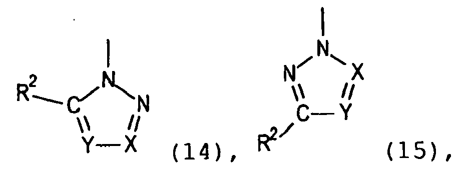 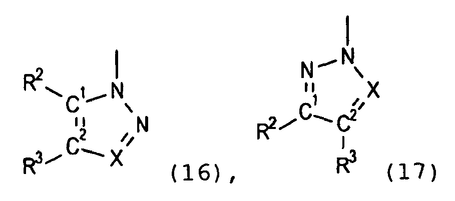 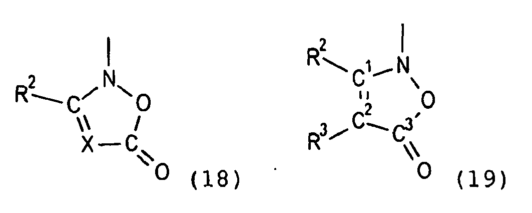
sowie deren Ester, Ether oder Salze ist,
und R¹, R², R³, R⁴, X und Y die zu den Formeln (1) bis (6) genannte Bedeutung haben.

11. Verfahren gemäß einem oder mehreren der Ansprüche 1 bis 10, **dadurch gekennzeichnet, daß** die nicht-proteinogene L-Aminosäure nach Beendigung des Verfahrens mittels bekannter Methoden isoliert wird.

12. Verfahren nach Anspruch 11 **dadurch gekennzeichnet, daß** die Isolierung mittels Filtration, Zentrifugation, Extraktion, Adsorption, Ionenaustauscher-Chromatographie, Präzipitation oder Kristallisation erfolgt.

## Claims

1. Process for preparing a non-proteinogenic L-amino acid by means of an enzymic biotransformation in which O-acetyl-L-serine is reacted with a nucleophilic compound, while using an O-acetyl-L-serine sulfhydrylase as catalyst, to give a non-proteinogenic L-amino acid, **characterized in that** the biotransformation is effected using resting microorganism cells which possess an O-Acetyl-L-serine sulfhydrylase activity and the process is carried out at a pH in the range between pH 6.0 and 6.99.

2. Process according to Claim 1, **characterized in that** CysM is used as the O-acetyl-L-serine sulfhydrylase.

3. Process according to Claim 1 or 2, **characterized in that** the O-acetylserine sulfhydrylase has a volume activity, A_{cys}, of at least 2 units/ml in the mixture.

4. Process according to one of Claims 1 to 3, **characterized in that** an O-acetyl-L-serine-containing fermenter broth is used as the O-acetyl-L-serine.

5. Process according to one of Claims 1 to 4, **characterized in that** the nucleophilic compound
H-S-, H-Se-, -N=N⁺=N⁻, -C≡N, 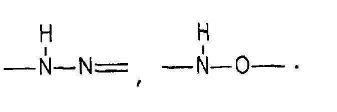 contains a radical selected from the group

6. Process according to Claim 5, **characterized in that** the nucleophilic compound is selected from the group of the thiosulfates, of the thiols of the general formula (1) :
H-S-R¹ (1)
where R¹ is a monovalent substituted or unsubstituted alkyl, alkoxy, aryl or heteroaryl radical having from 1 to 15 C atoms,
of the selenides, of the selenols of the general formula (2)
H-Se-R¹ (2)
where R¹ has the meaning given for formula (1),
of the azides, of the cyanides, of the azoles of the general formula (3) or (4): 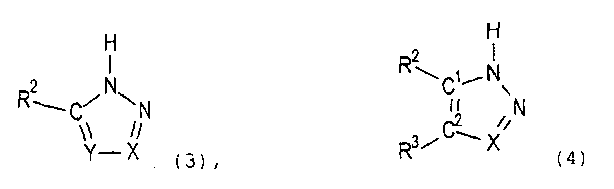 where X and Y are identical or different and are CR⁴ or N, and R⁴ is -H, -COOH, -OH, -NH₂, -NO₂⁻, -SH, -SO₃⁻, -F, -Cl, -Br, -I, C₁-C₅-alkylcarbonyl- and also their esters, amides or salts, or R¹, and R¹ has the meaning given for formula (1), and
where R² and R³ are identical or different and are R⁴ or where C¹ and C² in formula (4) are linked, in place of the substituents R² and R³, by means of a bridge [-CR⁵R⁶-]ₐ, where a is 1, 2, 3 or 4, to form a ring, where
R⁵ and R⁶ are identical or different and are R⁴, and one or more non-adjacent groups [-CR⁵R⁶-] can be replaced with oxygen, sulfur or an imino radical which is optionally substituted by C₁-C₅-alkyl, and two adjacent groups [-CR⁵R⁶-] can be replaced with a group [-CR⁵=CR⁶-] or with a group [-CR⁵=N-],
of the isoxazolinones of the general formula (5) or (6) : 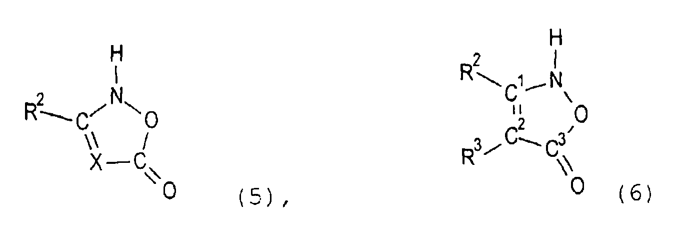 where X, R¹, R² and R³ have the meaning which has already been given, and
where C¹ and C² in formula (6) can be linked, in place of the substituents R² and R³, by means of a bridge as defined for formula (4) to form a ring.

7. Process according to Claim 5 or 6, **characterized in that** the nucleophilic compound is selected from the group 2-mercaptoethanol, 3-mercaptopropanol, 3-mercaptopropionic acid, 3-mercapto-1-propanesulfonic acid, mercaptoethanesulfonic acid, 2-mercaptoethylamine, thioglycolic acid, thiolactic acid, thioacetic acid, mercaptosuccinic acid, mercaptopyruvic acid, dithiothreitol, dithioerythritol, 1-thioglycerol, thiophenol, 4-fluorothiophenol, 4-chlorothiophenol, 4-mercaptophenol, p-thiocresol, 5-thio-2-nitrobenzoic acid, 2-mercaptothiazole, 2-mercaptothiazoline, 2-mercaptoimidazole, 3-mercapto-1,2,4-triazole, 2-thiophenethiol, 2-mercaptopyridine, 2-mercaptopyrimidine, 2-thiocytosine, 2-mercaptonicotinic acid, 2-mercapto-1-methylimidazole, 2-mercaptobenzothia-zole, 2-mercaptobenzoxazole, 6-mercaptopurine, sodium thiosulfate, potassium thiosulfate, ammonium thiosulfate, sodium azide, potassium azide, ammonium azide, potassium cyanide, sodium cyanide, ammonium cyanide, methylselenol, ethylselenol, propylselenol, phenylselenol, 1,2-pyrazole, 3-methylpyrazole, 4-methylpyrazole, 3,5-dimethylpyrazole, 3-aminopyrazole, 4-aminopyrazole, pyrazole-4-carboxylic acid, pyrazole-3,5-dicarboxylic acid, 1,2,3-triazole, 1,2,4-triazole, 3-amino-1,2,4-triazole, 1,2,3,4-tetrazole, indazole, indazole-3-carboxylic acid, indazole-5-carboxylic acid, 5-aminoindazole, benzotriazole, benzotriazole-5-carboxylic acid, 5-aminobenzotriazole, aminopyrazolopyrimidine, 8-azaguanine, 8-azaadenine, isoxazolin-5-one, 3-methylisoxazolin-5-one, 4-methylisoxazolin-5-one, 4,5-dimethylisoxazolin-2-one and 1,2,4-oxadiazolidine-3,5-dione.

8. Process according to one of Claims 1 to 7, **characterized in that** the concentration of the nucleophilic compound is selected such that the compound is present in a concentration which is equimolar with that of O-acetyl-L-serine.

9. Process according to one of Claims 1 to 8, which is carried out at a temperature between 5°C and 70°C.

10. Process according to one of Claims 1 to 9, **characterized in that** the unnatural L-amino acid is selected from the group of the amino acids of the general formula (7) in the L configuration 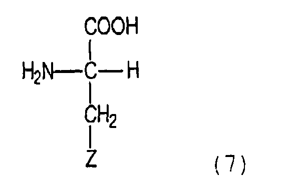 where Z is a monovalent radical in accordance with one of the formulae (8) to (19)
-S-SO₃⁻ (8),
-S-R¹ (9),
-Se-H (10),
-Se-R¹ (11),
-N=N⁺=N⁻ (12),
-C≡N (13), 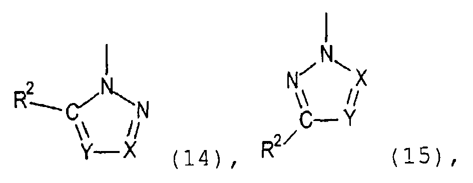 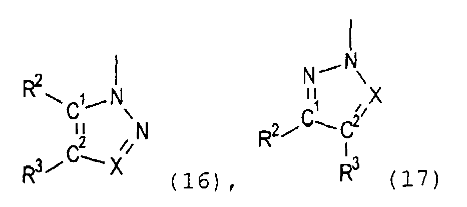 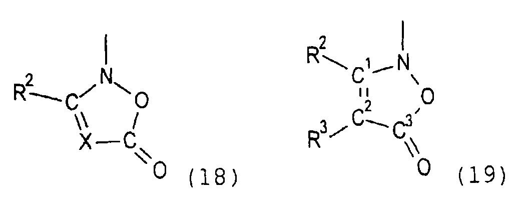
and the esters, ethers or salts thereof,
and R¹, R², R³, R⁴ , X and Y have the meaning given for the formulae (1) to (6).

11. Process according to one or more of Claims 1 to 10, **characterized in that** known methods are used to isolate the non-proteinogenic L-amino acid after the process has been terminated.

12. Process according to Claim 11, **characterized in that** the isolation is effected by means of filtration, centrifugation, extraction, adsorption, ion exchange chromatography, precipitation or crystallization.

## Revendications

1. Procédé pour la préparation d'un acide L-aminé non protéinogène au moyen d'une biotransformation enzymatique dans laquelle on transforme de l'O-acétyl-L-sérine avec un composé nucléophile sous une catalyse par une O-acétyl-L-sérine-sulfhydrylase en un acide L-aminé non protéinogène, **caractérisé en ce que** la biotransformation est réalisée en utilisant des cellules de microorganismes au repos, qui possèdent une activité d'O-acétyl-L-sérine-sulfhydrylase et le procédé est réalisé à un pH dans la plage entre pH 6,0 et 6,99.

2. Procédé selon la revendication 1, **caractérisé en ce qu'**on utilise comme O-acétyl-L-sérine-sulfhydrylase de la CysM.

3. Procédé selon la revendication 1 ou 2, **caractérisé en ce que** l'O-acétyl-sérine-sulfhydrylase présente une activité volumique A_{Cys} d'au moins 2 unités/ml dans la charge.

4. Procédé selon l'une quelconque des revendications 1 à 3, **caractérisé en ce qu'**on utilise comme O-acétyl-L-sérine un bouillon de fermentation contenant de l'O-acétyl-L-sérine.

5. Procédé selon l'une quelconque des revendications 1 à 4, **caractérisé en ce que** le composé nucléophile contient un radical choisi dans le groupe
H-S- H-Se- -N=N=N-, -C≡N,

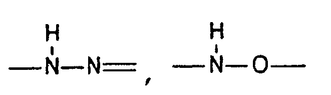

6. Procédé selon la revendication 5, **caractérisé en ce que** le composé nucléophile est choisi dans le groupe des thiosulfates, des thiols de formule générale (1) :
H-S-R¹ (1)
dans laquelle R¹ signifie un radical alkyle, alcoxy, aryle ou hétéroaryle monovalent, substitué ou non substitué, comprenant 1 à 15 atomes de carbone,
des séléniures, des sélénols de formule générale (2)
H- Se - R¹ (2)
dans laquelle R¹ présente la signification mentionnée pour la formule (1),
des azides, des cyanures, des azoles de formule générale (3) ou (4) : 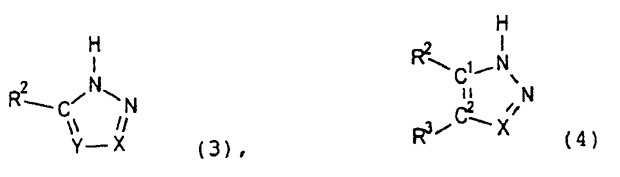 dans laquelle X et Y sont identiques ou différents et signifient CR⁴ ou N et R⁴ signifie -H, -COOH, -OH, -NH₂, -NO₂⁻, -SH, -SO₃⁻, -F, -Cl, -Br, -I, (alkyle en C₁ à C₅)carbonyle ainsi que leurs esters, amides ou sels ou R¹ et R¹ a la signification mentionnée pour la formule (1) et
dans laquelle R² et R³ sont identiques ou différents et signifient R⁴ ou dans laquelle C¹ et C² dans la formule (4), au lieu de présenter les substituants R² et R³, sont reliés au moyen d'un pont [-CR⁵R⁶-]ₐ avec a = 1, 2, 3 ou 4 en un cycle, où
R⁵ et R⁶ sont identiques ou différents et signifient R⁴ et un ou plusieurs groupes [-CR⁵R⁶-] non adjacents peuvent être remplacés par oxygène, souffre ou un radical imino le cas échéant substitué par alkyle en C₁ à C₅ et
deux groupes [-CR⁵R⁶-] adjacents peuvent être remplacés par un groupe [-CR⁵=CR⁶-] ou par un groupe [-CR⁵=N-],
des isoxazolinones de formule générale (5) ou (6): 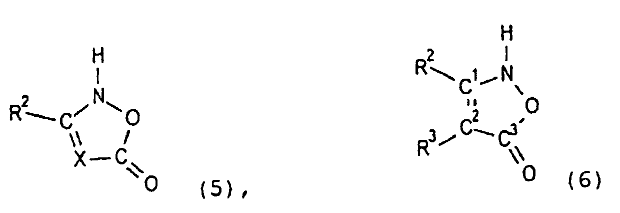 dans laquelle X, R¹, R² et R³ ont la signification déjà mentionnée
et
où C¹ et C² dans la formule (6), au lieu de présenter les substituants R² et R³, peuvent être reliés au moyen d'un pont tel que défini pour la formule (4) en un cycle.

7. Procédé selon la revendication 5 ou 6, **caractérisé en ce que** le composé nucléophile est choisi dans le groupe formé par le 2-mercaptoéthanol, le 3-mercaptopropanol, l'acide 3-mercaptopropionique, l'acide 3-mercapto-1-propanesulfonique, l'acide mercaptoéthanesulfonique, la 2-mercaptoéthylamine, l'acide thioglycolique, l'acide thiolactique, l'acide thioacétique, l'acide mercaptosuccinique, l'acide mercaptopyruvique, le dithiothréitol, le dithioérythritol, le 1-thioglycérol, le thiophénol, le 4-fluorothiophénol, le 4-chlorothiophénol, le 4-mercaptophénol, le p-thiocrésol, l'acide 5-thio-2-nitrobenzoïque, le 2-mercaptothiazole, la 2-mercaptothiazoline, le 2-mercaptoimidazole, le 3-mercapto-1,2,4-triazole, le 2-thiophènethiol, la 2-mercaptopyridine, la 2-mercaptopyrimidine, la 2-thiocytosine, l'acide 2-mercaptonicotinique, le 2-mercapto-1-méthylimidazole, le 2-mercaptobenzothiazole, le 2-mercaptobenzoxazole, la 6-mercaptopurine, le thiosulfate de sodium, le thiosulfate de potassium, le thiosulfate d'ammonium, l'azide de sodium, l'azide de potassium, l'azide d'ammonium, le cyanure de potassium, le cyanure de sodium, le cyanure d'ammonium, le méthylsélénol, l'éthylsélénol, le propylsélénol, le phénylsélénol, le 1,2-pyrazole, le 3-méthylpyrazole, le 4-méthylpyrazole, le 3,5-diméthylpyrazole, le 3-aminopyrazole, le 4-aminopyrazole, l'acide pyrazole-4-carboxylique, l'acide pyrazole-3,5-dicarboxylique, le 1,2,3-triazole, le 1,2,4-triazole, le 3-amino-1,2,4-triazole, le 1,2,3,4-tétrazole, l'indazole, l'acide indazole-3-carboxylique, l'acide indazole-5-carboxylique, le 5-aminoindazole, le benzotriazole, l'acide benzotriazole-5-carboxylique, le 5-aminobenzotriazole, l'aminopyrazolopyrimidine, la 8-azaguanine, la 8-azaadénine, l'isoxazolin-5-one, la 3-méthylisoxazolin-5-one, la 4-méthylisoxazolin-5-one, la 4,5-diméthylisoxazolin-2-one, la 1,2,4-oxadiazolidine-3,5-dione.

8. Procédé selon l'une quelconque des revendications 1 à 7, **caractérisé en ce que** la concentration en composé nucléophile est choisie de telle manière qu'il se trouve dans une concentration équimolaire avec l'O-acétyl-L-sérine.

9. Procédé selon l'une quelconque des revendications 1 à 8, **caractérisé en ce qu'**il est réalisé à une température entre 5°C et 70°C

10. Procédé selon l'une quelconque des revendications 1 à 9, **caractérisé en ce que** l'acide L-aminé non naturel est choisi dans le groupe des acides aminés de formule générale (7) dans la configuration L 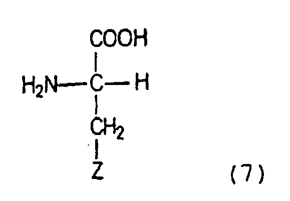 dans laquelle Z représente un radical monovalent selon l'une des formules (8) à (19)
-S-SO₃⁻ (8),
-S-R¹ (9),
-Se-H (10),
-Se-R¹ (11),
-N=N⁺=N⁻ (12),
-C≡N (13), 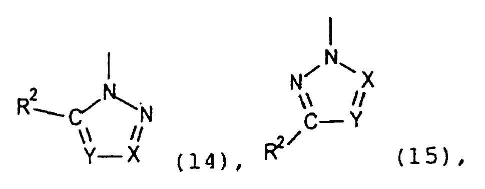 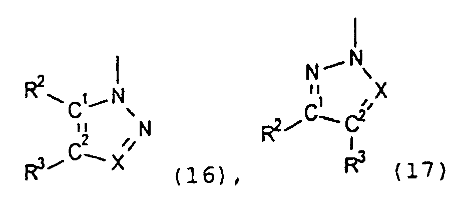 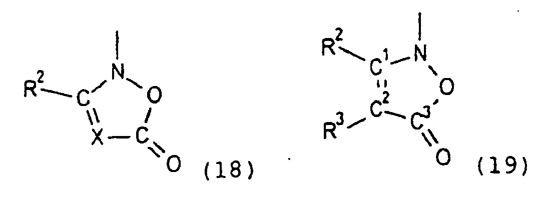
ainsi que leurs esters, éthers ou sels,
et R¹, R², R³, R⁴, X et Y présentent la signification indiquée pour les formules (1) à (6).

11. Procédé selon l'une ou plusieurs des revendications 1 à 10, **caractérisé en ce que** l'acide L-aminé non protéinogène est isolé à la fin du procédé par des procédés connus.

12. Procédé selon la revendication 11, **caractérisé en ce que** l'isolement est réalisé par filtration, centrifugation, extraction, adsorption, chromatographie sur un échangeur d'ions, précipitation ou cristallisation.
